# EUROPEAN PATENT APPLICATION

(11) **EP 2 573 168 A1**
(43) Date of publication of application: **27.03.2013**
(21) Application number: 10849179.6
(22) Date of filing: 22.07.2010
(51) Int. Cl.: C12N 5/0775, C12N 5/079, C12N 5/02, A61K 35/12, A61K 35/28, A61K 35/50, A61P 25/00, A61P 25/16, A61P 25/28, C12R 1/91

(54) **METHODS FOR PRODUCING NERVE CELLS FROM STEM CELLS, NERVE CELLS AND USES THEREOF**

(71) Applicant: Affiliated Hospital Of Ningxia Medical University, Yinchuan, Ningxia 750004 (CN)
(72) Inventor: YANG, Yinxue, Yinchuan Ningxia 750004 (CN); WEI, Jun, Yinchuan Ningxia 750004 (CN); LI, Yukui, Yinchuan Ningxia 750004 (CN); WANG, Libin, Yinchuan Ningxia 750004 (CN); MA, Lijun, Yinchuan Ningxia 750004 (CN); LIU, Ting, Yinchuan Ningxia 750004 (CN); MA, Xiaona, Yinchuan Ningxia 750004 (CN); FAN, Heng, Yinchuan Ningxia 750004 (CN); ZHU, Yongzhao, Yinchuan Ningxia 750004 (CN); YE, Ping, Yinchuan Ningxia 750004 (CN); JIN, Yiran, Yinchuan Ningxia 750004 (CN)
(74) Representative: O'Neill, Michelle
(86) International application number: PCT/CN2010/001114
(87) International publication number: WO 2012/009830

(57) **Abstract**

The present invention belongs to the field of cell transplantation. Particularly, the present invention provides a method of producing neurons for treating injuries with the loss of neuron function from stem cells, wherein the stem cell is a human derived mesenchymal stem cell, preferably human derived placental mesenchymal stem cell, bone marrow mesenchymal stem cell, adipose mesenchymal stem cell and liver mesenchymal stem cell. The present invention also provides the uses of said method and the nerve cells produced by said method in the preparation of medicines for treating injuries with the loss of neuron function and in the treatment of injuries with the loss of neuron function. The present invention further provides a method of treating injuries with the loss of neuron function.

## Description

### Technical Field

The present invention belongs to the field of cell transplantation. Particularly, the present invention is related to a method of producing neurons that may be used to treat injuries with the loss of neuron function, and the use of said method and the neurons obtained in the treatment of injuries with the loss of neuron function. The present invention is further related to a method of treating injuries with the loss of neuron function.

### Background of the Invention

Parkinson's disease is a degenerative disease of the central nervous system, which is usually caused by the degeneration or lesions of dopamine producing neurons in the midbrain. The clinical and pathological manifestation of Parkinson's disease is the lack of dopamine. Theoretically, the symptoms of Parkinson's disease may be alleviated or treated by supplementing dopamine. However, the therapeutic medicines now widely used in clinical are the precursors of dopamine, L-DOPA, because the dopamine is not able to across the blood-brain barrier. However, after the injection of L-DOPA, most of them will be metabolized in the body before reaching the central nervous system, with only less than 5% of the effective dose of L-DOPA reaching the effective part of the central nervous system. The metabolism of the remaining 95% of L-DOPA causes a variety of side effects, and further reduces the production of dopamine in the body from a feedback mechanism. Therefore, the long-term administration of L-DOPA has been proved to have no therapeutic effect for Parkinson's disease.

Another process tried in the clinical is to use dopamine analogs, such as bromocriptine, pergolide or perxole and the like, which may bind to the dopamine acceptors in the central neuron, and therefore simulating the effect of dopamine. However, research shows that such dopamine analogs usually make the dopamine acceptors gradually lose the sensitivity to dopamine and the analogs thereof, therefore the long-term administration of such dopamine analogs may aggravate rather than reduce the symptoms of Parkinson's disease.

Due to the reasons above, currently Parkinson's disease could only be clinically reduced, rather than cured.

Spinal cord injury is another disease with the loss of central neural function, and usually caused by sport injury, local ischemic injury, nerve degenerative disease and myelitis with the clinical symptom of loss of the motor neuron function, thus leading to part or all of the body below the injured area paralysis. Currently there is no therapeutic method to recover the function of the injured motor neuron, so this injury could not be cured at present.

Recent researches show that cell transplant may provide an effective method for treating Parkinson's disease and spinal cord injury (Freed et al., 2001; Kumar et al., 2009). Freed et al. transplanted the cells taken from aborted infants' central nervous tissue into the midbrain of patients with Parkinson's disease. It was found upon one-year research that the symptoms of Parkinson's disease had mild improvement in the group of younger patients. The cells transplanted in this research could secret nerve factor such as dopamine as proved in *in vitro* cultures. However, it is impossible to control the amount of effective cells in such cell transplantations, and so it is unable to control the level of dopamine because the fetal central nervous tissue is a mixture of many different types of cells with the unknown proportion of the nerve factor secreting cells. Therefore, this research shows nerve factor secreting cells may be used in the treatment of Parkinson's disease by means of cell transplantation, but the method used does not get the expected results.

Kumar et al. transplanted bone marrow monocytes from spinal cord injuries patients back to corresponding patients, and found one third of the patients had shown slightly ameliorated symptoms. A one-year follow-up study found that no patients receiving the cell transplantation suffered from obvious side effects. The author believes the environment at the sites of the spinal cord injuries induced the bone marrow cells to differentiate into nerve cells and thus the symptom is ameliorated. However, only a tiny part of the bone marrow monocytes possess the ability to differentiate into nerve cells, so Kumar's method is not able to control the number of effective cells in the transplanted cells. Moreover, the attachment of mesenchymal cells in the bone marrow cells may increase the deposit of some glycoproteins at the injury sites, and thus affects the recovery of nerve injuries.

In order to overcome the problem of selecting effective nerve factor producing cells and motor neurons in clinical cell transplantation and controlling the mount of the same, many researches has tried to produce nerve factor producing cells and motor neurons from embryonic stem cells. Kawasaki et al. reported that human embryonic stem cells could be differentiated into dopamine producing cells and motor neurons using mouse PA6 cell as nutrient cells. Using such method, human embryonic stem cells are cultured on a cell nutrient layer formed by PA6 cells to differentiate the embryonic stem cells into ectodermal cells. Then proper nerve factors, like Wnt3 (Wingless family number 3) or Shh (Sonic hedgehog) may be employed to further differentiate the ectodermal cells into dopamine secreting cells and motor neurons. The defect of such method is that it is required to culture the human embryonic stem cells with the nutrient cells of mice origin, and the cells obtained by such method thus are not suitable for clinical use because of contamination caused by animal original proteins and possible animal original pathogeny.

In order to solve the problems described above, an existing invention (US Patent Application No. 20030162290) provided a method without using nutrient layer cells of an animal origin. In this invention, leukocyte inhibition factor (LIF) and fibroblast growth factor are used to induce the embryonic stem cells into embryoid bodies, and then the embryoid body cells are differentiated into nerve cells with nicotinamide and insulin. In this method embryonic stem cells are successfully differentiated into cells with neuron functions, however it does not produce cells with neuron functions from other cells, like adult stem cells. Limitations of the cells for clinical use produced by such method are that the cells of embryonic stem cell origin are all allosomal cells and thus immunological rejection and corresponding immunological reaction syndromes may occur after the cell transplantation. Also, the use of embryonic stem cells will along with well-accepted ethical issues.

Therefore, currently one of the problems to be solved on the treatment of stem cells for Parkinson's disease and spinal cord injury is how to produce the neurons with properties of secreting nerve factors and properties of motor neurons from adult stem cells.

### Summary of the Invention

Aiming at the deficiency of the prior art, the main object of the present invention is to provide a method of producing neurons with properties of secreting nerve factors and properties of motor neurons from mesenchymal stem cells including human derived placental mesenchymal stem cells, bone marrow mesenchymal stem cells, adipose mesenchymal stem cells, liver mesenchymal stem cells and other sources of mesenchymal stem cells. In such method animal cells are not used to provide an inducing environment, and cells with neuron functions may be produced from adult stem cells, therefore the method is more suitable for the production of cells for clinical application. Another aspect of the invention is to provide the obtained cells with neuron functions. In still another aspect of the invention is to provide the uses of said method and the cells produced by said method.

Technical solutions are provided below to achieve the above object.

In one aspect, the present invention provides a method of producing the cells with neuron functions from stem cells, the method comprises the following steps:
1) culturing the obtained stem cells in a medium with high serum content;
2) transferring the cells cultured in the first step into a medium having low serum content and containing cell growth factors and neurotrophic factors;
3) transferring the cells cultured in the second step into a differentiation medium and continue to culture the cells;
4) transferring the cells cultured in the third step into a medium containing nerve cell maturation factors and continue to culture the cells;
5) transferring the cells cultured in the fourth step into a medium containing nerve factors.

In the method described above, the stem cells used are preferably human derived placental mesenchymal stem cells and marrow mesenchymal stem cells, and other stem cells suitable for the method of the present invention include adult derived adipose mesenchymal stem cells, adult liver mesenchymal stem cells and other sources of adult mesenchymal stem cells; and further preferably, the stem cells are placental mesenchymal stem cells.

The obtained cells with neuron functions are neurons with the properties of secreting nerve factor and properties of motor neurons.

Preferably, the medium used in Step 1) is DMEM supplemented with 15-25% serum, wherein the serum is preferably 20% FBS; the culture time of the cells is 8-24 hours, preferably 16 hours. Preferably, the medium used in Step 2) is an initial differentiation medium, which is a DMEM/F12 mixture medium supplemented with 0.1-0.5% serum, and the medium comprises equal content of N2 mixed cofactor (100mg/L of transferrin, 5mg/L of insulin, 6.3ug/L of progestational hormone, 16mg/L of putrescine, 5.2ug/L of selenite), 5-15 ng/ml of fibroblast growth factor (FGF) and 5-15ng/ml of endothelial growth factor (EGF) and the culture time of the cells is 8-24 hours; said initial differentiation medium is preferably a DMFM/F12 mixture medium supplemented with 0.2% of FBS, and contains equal content of N2 mixed cofactor, 2 µg/ml of heparin, 1% MEM, 10 ng/ml of bFGF (basic FGF) and 10 ng/ml of EGF, and the culture time of the cells is preferably 24 hours.

Preferably, the medium used in Step 3) is a differentiation medium, which is a DMEM/F12 mixture medium supplemented with 0.1-0.5% serum, and contains equal content of N2 mixed cofactor and 50-500 of µg/ml embryonic brain protein, and the culture time of the cells is 3-15 days; the differentiation medium is preferably a DMEM/F12 mixture medium supplemented with 0.2% FBS, and contains equal content of N2 mixed cofactor, 2 µg/ml heparin, 1% MEM and 200 µg/ml of embryonic brain protein, and the culture time of the cells is preferably 6 days.

Wherein the embryonic brain protein used in Step 3) is the total protein from embryonic brain tissue, and typical embryonic brain proteins are commercially available.

Preferably, the medium used in Step 4) is a DMEM/F12 mixture medium supplemented with 0.1-0.5% serum, and contains equal content of N2 mixed cofactor, 10-50 nM of retinoic acid (RA) and 100-1000ng/mL of Sonic hedgehog (Shh), and the culture time of the cells is 2-5 days; this medium is preferably a DMEM/F12 mixture medium supplemented with 0.2% FBS, and contains 1% of N2 mixed cofactor, 2 µg/ml of heparin, 20nM of RA, and 500 ng/ml of Shh, and the culture time of the cells is preferably 3 days.

Preferably, the medium used in Step 5) is a DMEM/F12 mixture medium supplemented with 5-15% serum, and contains 5-20 ng/ml of brain-derived neurotrophic factor (BDNF), 10-50 ng/ml of glial cell-derived neurotrophic factor (GDNF) and 20-50 ng/ml of insulinoid growth factor (IGF), and the culture time of the cells is 5-10 days; preferably, the medium is a DMEM/F12 mixture medium supplemented with 10% FBS, and contains 10 ng/ml of BDNF, 20 ng/ml of GDNF and 50 ng/ml of IGF-II, and the culture time of the cells is preferably 7 days.

In the method mentioned above, the serum used is selected from human umbilical cord blood serum or fetal bovine serum; the condition of the incubator is 5% carbon dioxide and 95% air.

In another aspect, the present invention provides cells with neuron functions as prepared by the methods above, wherein the cells are preferably nerve cells capable of expressing neuron specific enolase (NSE) and neurofilament protein-M (NF-M), as well as secreting neurotrophic factor (NT) and brain derived neurotrophic factor (BDNF); further preferably, the cells are neurons which are capable of expressing neuron specific enolase (NSE), glial fibrillary acidic protein (GFAP), growth related protein factor 43 (GAP-43), and neurofilament protein-M (NF-M), as well as secreting neurotrophic factor-3 (NT-3), neurotrophic factor-4 (NT-4), and brain derived neurotrophic factor (BDNF).

In another aspect, the present invention provides uses of the above mentioned method and the cells with neuron functions produced by the method in the preparation of medicines to treat injuries with the loss of neuron function.

Preferably, the cells with neuron functions are neurons expressing neuron specific enolase (NSE) and neurofilament protein-M (NF-M), as well as secreting neurotrophic factor (NT) and brain derived neurotrophic factor (BDNF); the injuries with the loss of neuron function are Parkinson's disease and spinal cord injury.

In addition, the present invention also provides a method of producing the cells with neuron functions and uses of the produced cells with neuron functions in the treatment of injuries with the loss of neuron function, which is preferably Parkinson's disease and spinal cord injury.

In another aspect, the present invention provides a method of treating injuries with the loss of neuron function, wherein the method comprises transplanting the cells with neuron functions produced by the above described method into the injury sites of patients with neurological diseases, and the neurological disease include, but not limited to Parkinson's disease and spinal cord injury; preferably, the cells with neuron functions express neuron specific enolase (NSE), neurofilament protein-M (NF-M), and secret neurotrophic factor (NT) and brain derived neurotrophic factor (BDNF).

A detailed description of the invention is as follows:
One object of the present invention is to provide a method of producing cells with neuron functions from mesenchymal stem cells, especially a method of producing cells with the properties of secreting nerve factors and the properties of motor neuron. Another object of the present invention is to provide nerve cells with the properties of secreting nerve factors and the properties of motor neuron, wherein the cells may be used for the treatment of Parkinson's disease and spinal cord injury. Still another object of the invention is to provide a method of treating Parkinson's disease and spinal cord injury, which recovers the neuron functions by transplanting the cells with neuron functions.

Now the present invention is described combined with the objects of the invention.

The embodiment of the inventive contents described above may comprises the steps as follows:
1. Human derived mesenchymal stem cells are cultured in a medium with high serum content for 16 hours;
2. The cells obtained in the first step are cultured in a medium having low serum content and containing cell growth factors and neurotrophic factors for 16 hours;
3. The cells obtained in the second step are cultured in a differentiation medium for 6 days;
4. The cells obtained in the third step are cultured in a medium containing neuron maturation factors for 3 days;
5. The cells obtained in the fourth step are cultured in a medium containing nerve factors for 7 days;
6. The cells obtained in the fifth step are transplanted into the midbrain region of the patients with Parkinson's disease, and the injury sites of patients with spinal cord injuryto recover the lost neuron functions of the patients.

In the first step of the invention, the cells to be differentiated may be human derived various types of mesenhymal stem cells, which include placental mesenhymal stem cells, bone marrow mesenhymal stem cells, adipose msesnhymal stem cells, liver mesenhymal stem cells, and other sources of adult mesenhymal stem cells. The medium used is a standard DMEM (Dulbecco's Modified Eagle Medium) medium, and the serum used may be human umbilical cord blood serum or fetal bovine serum (FBS), and the serum content is 15-25%. The condition of the incubator is 5% carbon dioxide and 95% air. This incubator condition is suitable for all the following steps.

In the second step, the cells obtained in the first step are transferred into a medium with low serum content to induce the initiation of the differentiation, and the medium used is a standard DMEM/F12 (DMEM/Nutrient Mix 12) mixture medium. The serum content is 0.1-0.5%. Moreover, the medium comprises equal content of N2 mixed cofactor, 5-15ng/ml of fibroblast growth factor (FGF), and 5-15ng/ml of endothelial growth factor (EFG).

In the third step, the cells obtained in the second step are transferred into a differentiation medium. The main component for induction in such medium is the embryonic brain protein, wherein 50-500µg/ml of pure protein is employed. The basic medium used is the same as that in the second step except that such medium does not contain FGF or EGF.

In this step the medium is refreshed every two days, and the cells are cultured for 3-15 days.

In the fourth step, the cells obtained in the third step are converted to functioning cells in a medium containing neuron maturation factor. Neuron maturation factor include 20nM of retinoic acid and 500ng/ml of hedgehog homologous protein. The basic medium used here is the same as that in the third step, except that embryonic brain proteins are not included.

In the fifth step, the cells obtained in the fourth step are proliferated in a medium for neuron cells. The medium employed is a standard DMEM/F12 mixture medium, which comprises 5-15% serum, 5-20ng/mlof brain-derived neurotrophic factor (BDNF), 10-50ng/ml of glial cell-derived neurotrophic factor (GDNF) and 20-50 ng/ml of insulinoid growth factor (IGF).

The cells obtained in the method described above show the morphology of fibroblast cells (Fig.1), express nerve cells specific proteins including neuron specific enolase (NSE), nestin, growth associated factor -43 (GAP-43), glial fibrillary acidic protein (GFAP) and neurofilament protein (NF-M) (Fig. 2), and secret neuron specific protein factors, such as neurotrophic factor-3 (NT3), neurotrophic factor-4 (NT4) and brain derived neurotrophic factors (BDNF) (Fig. 3). These results suggest the cells produced by the method of the present invention possess characteristics of neuronal functions.

The present invention further provides one method of treating injuries with the loss of neuron function by using the cells obtained above, which comprises transplanting the cells obtained in the present invention into the injury sites of the patients with neurological diseases. The diseases treated include but not limited to Parkinson's disease and spinal cord injury. The method for cell transplantation may be the same as the current clinical methods of cell transplantation for the same diseases, like the method by Freed et al. to transplant the dopamine secreting cells to treat Parkinson's disease, or the method by Kumar et al. to transplant bone marrow cells to treat spinal cord injuries.

Advantages of the invention include:
1. The cells taken from animal are not used to provide an inducing environment in the method of the present invention during producing cells with neuron functions, so excluding contamination of the animal origin proteins and possible animal pathogeny, which make the cells obtained by this method more suitable for clinical applications;
2. Autogenic stem cells may be used to produce cells with neuron functions in the method of the invention, and thus avoiding possible immunologic rejections and corresponding immunologic reaction syndromes;
3. Adult stem cells may be used to produce cells with neuron functions for clinical application in method of the present invention, avoiding well accepted ethical issues along with embryonic stem cells.

### Description of the Drawings

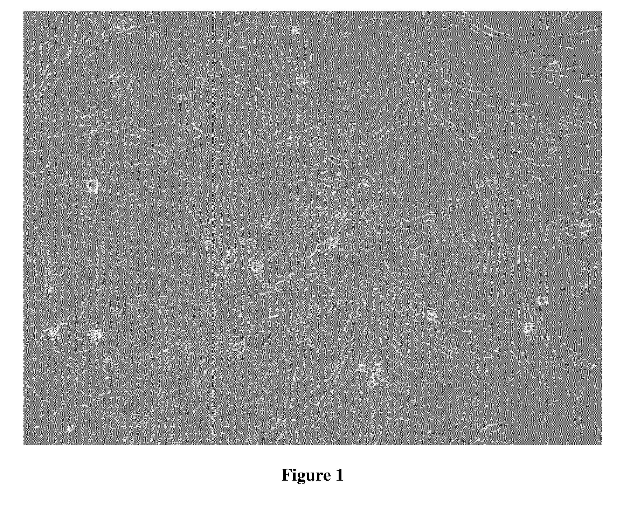

Fig. 1 represents the nerve cells induced from human placenta-derived mesenchymal cell.

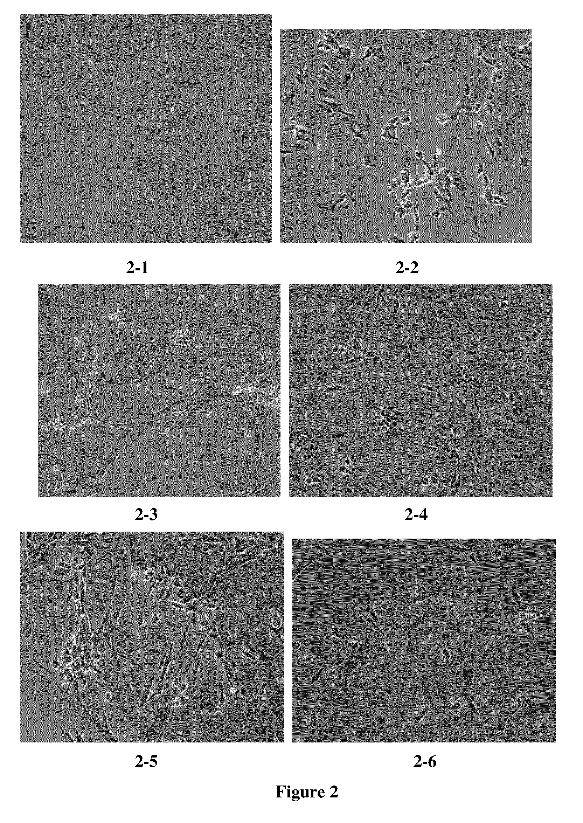

Fig. 2 represents the nerve cells induced from human placenta-derived mesenchymal cell express nerve cell specific proteins. Wherein:
   Fig. 2-1 is un-induced human placenta-derived mesenchymal cells (200×), wherein the staining for neurocytokine is negative;
   Fig. 2-2 is the nerve cells induced from placenta-derived mesenchymal cells (200×) wherein the brown-yellow color is the staining for Nestin;
   Fig. 2-3 is the nerve cells induced from placenta-derived mesenchymal cells (200×), wherein the brown-yellow color is the staining for NSE;
   Fig. 2-4 is the nerve cells induced from placenta-derived mesenchymal cells (200×), wherein the brown-yellow color is the staining for GFAP;
   Fig. 2-5 is the nerve cells induced from placenta-derived mesenchymal cells (200×), wherein the brown-yellow color is the staining for GAP-43;
   Fig. 2-6 is the nerve cells induced from placenta-derived mesenchymal cells (200×), wherein the brown-yellow color is the staining for NF-M;

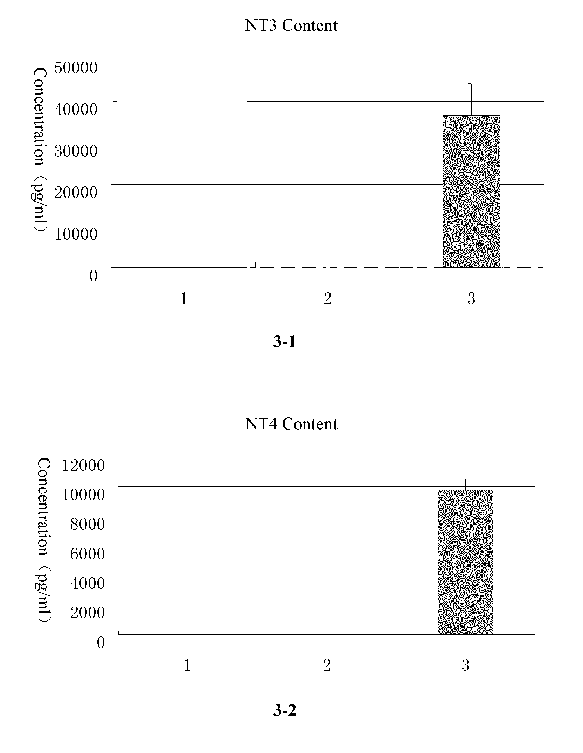

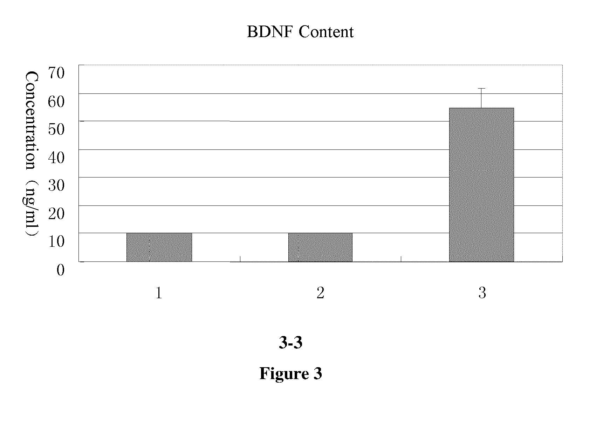

Fig. 3 represents the nerve cells induced from placenta-derived mesenchymal cells secret nerve factors and neurotrophic factors.
   Fig. 3-1 is the secret of neurotrophic factor-3 (NT-3) protein determined by ELISA
      1. The control medium, on which the cells have not been cultured, the concentration of NT-3 is 0pg/ml;
      2. The medium of cells in the control group (un-induced human placenta-derived mesenchymal stem cells), the concentration of NT-3 is 0pg/ml;
      3. The medium of cells after inducing with embryonic brain proteins, the concentration of NT-3 is 36645pg/ml.
   Fig. 3-2 is the secret of neurotrophic factor-4 (NT-4) protein determined by ELISA, wherein:
      1. The control medium, on which the cells have not been cultured, the concentration of NT-4 is 0pg/ml;
      2. The medium of cells in the control group (un-induced human placenta-derived mesenchymal stem cells), the concentration of NT-4 is 0 pg/ml;
      3. The medium of cells after inducing with embryonic brain proteins, the concentration of NT-4 is 9789 pg/ml.
   Fig. 3-3 is the secret of brain-derived nerve factor (BDNF) determined by ELISA, wherein:
      1. The control medium, on which the cells have not been cultured, the concentration of BDNF is 10ng/ml;
      2. The medium of cells in the control group (un-induced human placenta-derived mesenchymal stem cells), the concentration of BDNF is 10ng/ml;
      3. The medium of cells after inducing with embryonic brain proteins, the concentration of BDNF is 55ng/ml.

### Best mode for carrying out the invention

Following examples are used to assist the readers to understand the context of the invention rather than limiting the scope of the invention. For example, in the following examples human placental mesenchymal stem cells are used as the cells to be differentiated, but it is not difficult to understand for readers skilled in the art that other sources of mesenchymal stem cells including bone marrow mesenchymal stem cells, adipose mesenchymal stem cells, liver mesenchymal stem cells and so on are also suitable for the present invention.

### Example 1: Isolating human placental mesenchymal stem cells

The placentas used in this experiment are term placentas of healthy parturients, and obtained from the obstetric clinical in the Affiliated Hospital of Ningxia Medical University. Donors donate term placentas voluntarily and signed informed consents. Under sterile conditions, around 20g of fresh human placenta tissue is cut off from a placenta after delivery. The tissue is kept in a 50ml centrifuge tube with 20ml solution of 1% human umbilical cord blood serum and 1% penicillin/streptomycin (Invitrogen, Product code:15140122) . The placenta tissue in the protection solution above is transferred to the cGMP lab within 30 minutes. The tissue is washed three times in a PBS solution containing 1% penicillin/streptomycin (Invitrogen, Product code: 14040133) before processing.

Nondeciduate placentas are divided from the placenta tissue with a sterile surgical scissors, washed three times in PBS, and cut into around 1mm³ of small pieces, and then digested with a combination of 270 Unit/ml collagenase IV (Invitrogen, 17104019) and 2.4 Unit/ml dispase II (Roche, Product code: 04942078001) under 37°C for 1 hour to isolate placental amniotic mesenchymal cells and placental chorionic mesenchymal cells. After digestion, the tissue residues in the digesting mixture are stood for precipitation for 30 seconds, and the middle layer of the cell suspension is then collected. The collected cell suspension is diluted with PBS in equal volume and centrifuged at 700g for 10 minutes, and then the supernatant is discarded and the precipitated cells are washed twice in PBS containing 1% human umbilical cord blood serum, followed by washed once again in DMEM medium containing 1% human umbilical cord blood serum.

### Example 2: Embryonic brain proteins induce the differentiation of mesenchymal stem cells into nerve cells.

The steps of inducing the differentiation of mesenchymal stem cells into nerve cells comprise:
1. The human placental mesenchymal stem cells (MSCs) obtained in Example 1 is cultured in a standard medium (DMEM+10% FBS) until the density reaches 80%.
2. The cells are digested with 200 U/ml collagenase IV (Invitrogen) under 37°C for 3-5 minutes to obtain individual cells.
3. The cells are transferred into incubation flask coated with collagen protein-IV (Invitrogen), added with DMEM medium and cultured for 24 hours.
4. The medium is replaced with fresh medium (DMEM + 20% FBS) and continue to culture the cells for 16 hours.
5. The cells are cultured in an initial differentiation medium for 16 hours, wherein the initial differentiation medium is a standard DMEM/F12 medium which comprises equal content of N2 mixed cofactor, 2ug/ml heparin (Heparin, Sigma), 1% minimal essential medium (MEM, Invitrogen), 0.2% FBS, 10ng/ml bFGF (R&D Systems) and 10ng/ml EGF (Gibco).
6. The cells are further cultured in the differentiation medium for 6 days, wherein the differentiation medium is an initial differentiation medium but does not contain FGF or EGF, and contains 200 ug/ml embryonic brain protein (purchased from Capital Biosciences, Rockville, USA, Product code: TCB-1977). The medium is refreshed with fresh differentiation medium every two days.
7. The cells are further cultured in the maturing medium for 3 days. The maturing medium is similar to the initial differentiation medium except that the maturing medium does not comprise FGF or EG, and comprises 20nM RA (Sigma) and 500ng/ml SHH (Biosource)
8. The cells are further cultured in a neuron medium for 7 days, wherein the neuron medium is similar to the initial differentiation medium except that the neuron medium comprises 10% FBS, 10ng/ml BDNF (Invitrogen), 20ng/ml GDNF (Sigma), 50ng/ml IGF-II (R&D Systems), and does not comprise FGF or EFG. During this stage the culture is refreshed with fresh medium every two days.

The nerve-like cells obtained after inducing differentiation is illustrated in Fig. 1.

### Example 3: Determination of nerve cell specific proteins

The determination steps include:
1. Cell attached coverslip: The cells obtained (experimental group) in Example 3 are transferred into a culture dish covered with coverslip at a density of 3×10⁵ and cultured in the neuron medium (same with the medium in Step 6 of Example 3). Also un-induced human placenta mesenchymal stem cells are cultured under the same conditions as a control group. In the following steps the experimental group and the control group are treated identically.
2. After 24 hours, all the coverslips with cells attached (nerve-like cells and control cells respectively) are washed with PBS three times, and each time for 5 minutes.
3. The coverslips are treated in a mixture solution of 30% H₂O₂ (1 part) and pure methanol (50 parts) for 30 minutes and then washed with distilled water twice.
4. Blocking buffer containing 5% bovine serum protein (BSA) is dripped on the cells attached on the coverslips, and the coverslips are maintained under room temperature for 20 minutes, and then the excess liquid is shaken off without washing.
5. The properly diluted primary antibody is added (each antibody is diluted according to the instructions of manufactures), wherein the antibody includes the primary antibodies against NSE, Nestin, GAP-43, GFAP, NF-M (Boster Co., Ltd., China), and the cells are cultured at 4°C overnight.
6. The cells are washed with PBS (pH 7.2-7.6) three times, each time for 2 minutes.
7. The secondary antibody is added and the cells are incubated at 37°C for 20 minutes.
8. The cells are washed with PBS three times, each time for 2 minutes.
9. Streptavidin-biotin-peroxidase complex (SABC) is added, and the cells attached on the coverslips are cultured at 37°C for 20 minutes and then washed with PBS four times, each time for 5 minutes.
10. The coverslips are developed with diaminobenzidine (DAB) (DAB kit is employed, which is supplied by Boster Co., Ltd., China).
11. Dehydrate with graded ethanol, vitrification by dimethylbenzene, envelop by balata, and then observe cells on the slide under a microscope and take photos.

The determined results show the induced nerve-like cells are capable of expressing nerve cell specific proteins. (See Fig. 2)

### Example 4: Determination of neurocytokines and neurotrophic factors

The determination steps include:
1. The nerve cells obtained in Example 3 (experimental group) are cultured in the nerve cell medium (same with the medium in Step 6 of Example 3) with a density of 5×10⁵. Moreover, un-induced human placental mesenchymal cells are cultured under the same condition as the control group. In the following steps the experimental group and the control group are treated identically. The cell medium is collected after 48 hours and centrifuged at 12000r/min under 4°C for 5 minutes.
2. An enzyme linked immunosorbent assay (ELISA) kit (Shanghai Senxiong Technology Industry Co., Ltd) is balanced to room temperature (20-25°C).
3. Establishing Standard Curves: 8 standard wells are set with each 100µl of standard sample with gradient dilution added.
4. Adding the sample: into each of the testing wells 100µl assay sample is added.
5. The reaction plate is maintained under 37°C for 120 minutes.
6. Washing the plate: the reaction plate is washed sufficiently with a washing fluid for 4-6 times and dried on filter paper.
7. Into each well, 100µl working solution of primary antibody is added, and the reaction plate is sufficiently mixed and then maintained under 37°C for 60 minutes.
8. Washing the plate: the reaction plate is washed sufficiently with a washing fluid for 4-6 times and dried on filter paper.
9. Into each well, 100µl working solution of enzyme-labeled antibody is added, and the reaction plate is maintained under 37°C for 60 minutes.
10. Washing the plate: the reaction plate is washed sufficiently with a washing fluid for 4-6 times and dried on filter paper.
11. Into each well, 100µl substrate working solution is added, and the plate is maintained under 37°C in dark to react for 5-10 minutes.
12. 50µl stop solution is added into each well and mixed, and then the absorbance of the solution is measured at 492 nm.

### Reference

1. CURT R. FREED, PAUL E. GREENE, ROBERT E. BREEZE et al, 2001. TRANSPLANTATION OF EMBRYONIC DOPAMINE NEURONS FOR SEVERE PARKINSON'S DISEASE. N Engl J Med 2001;344:710-9.
2. Arachiman A. Kumau, Sankaran R. Kumar, Raghavachary Narayanan et al, 2009. Autologous Bone Marrow Derived Mononuclear Cell Therapy for Spinal Cord Injury: A Phase I/II Clinical Safety and Primary Efficacy Data. Experimental and Clinical Transplantation 4:241-248.
3. Hiroshi Kawasaki, Hirofumi Suemori, Kenji Mizuseki et al, 2002. Generation of dopaminergic neurons and pigmented epithelia from primate ES cells by stromal cell-derived inducing activity.PNAS 99(3):1580-1585.
4. Inoue, Kazutomo; Kim, Dohoon;; Gu, Yanjun; et al, 2003. Method for inducing differentiation of embryonic stem cells into functioning cells. United States Patent Application 20030162290.

## Claims

1. A method of producing cells with neuron functions from stem cells, which comprises the following steps:
1) culturing the obtained stem cells in a medium with high serum content;
2) transferring the cells cultured in the first step into a medium having low serum content and containing cell growth factors and neurotrophic factors;
3) transferring the cells cultured in the second step into a differentiation medium and continue to culture the cells;
4) transferring the cells cultured in the third step into a medium containing nerve cell maturation factors and continue to culture the cells;
5) transferring the cells cultured in the fourth step into a medium containing nerve factors and continue to culture the cells,
preferably, the stem cells are human derived mesenchymal stem cells; further preferably placental mesenchymal stem cells, bone marrow mesenhymal stem cells and adipose msesnhymal stem cells; more preferably placental mesenchymal stem cells;
preferably, the produced cells with neuron functions are nerve cells expressing neuron specific enolase (NSE) and neurofilament protein-M (NF-M), and secreting neurotrophic factor (NT) and brain derived neurotrophic factor (BDNF).

2. A method according to claim 1, wherein the medium used in Step 1) is DMEM supplemented with 15-25% of serum, wherein the serum is preferably 20% of FBS; the culture time of the cell is 8-24 hours, preferably 16 hours.

3. A method according to claim 1 or 2, wherein the medium used in Step 2) is an initial differentiation medium, which is a DMEM/F12 mixture medium supplemented with 0.1-0.5% of serum and comprises equal content of N2 mixed cofactor, 5-15 ng/ml of fibroblast growth factor (FGF) and 5-15ng/ml of endothelial growth factor (EGF) and the culture time of the cells is 8-24 hours; said initial differentiation medium is preferably a DMFM/F12 mixture medium supplemented with 0.2% of FBS, and contains 1% of N2 mixed cofactor, 2 µg/ml heparin, 1% MEM, 10 ng/ml of bFGF (basic FGF) and 10 ng/ml of EGF, and the culture time of the cells is preferably 16 hours.

4. A method according to any one of claims 1-3, wherein the medium used in Step 3) is a differentiation medium, which is a DMEM/F12 mixture medium supplemented with 0.1-0.5% serum, and contains equal content of N2 mixed cofactor and 50-500 µg/ml of embryonic brain protein, and the culture time of the cells is 3-15 days; the differentiation medium is preferably a DMEM/F12 mixture medium supplemented with 0.2% of FBS, and contains 1% of N2 mixed cofactor, 2 µg/ml heparin, 1% MEM and 200 µg/ml CNS protein, and the culture time of the cells is preferably 6 days.

5. A method according to any one of claims 1-4, wherein the medium used in Step 4) is a DMEM/F12 mixture medium supplemented with 0.1-0.5% of serum, and contains equal content of N2 mixed cofactor, 10-50 nM of retinoic acid (RA) and 100-1000ng/mL of Sonic hedgehog (Shh), and the culture time of the cells is 2-5 days; said medium is preferably a DMEM/F12 mixture medium supplemented with 0.2% of FBS, and contains 1% of N2 mixed cofactor, 2 µg/ml of heparin, 20nM of RA, and 500 ng/ml of Shh, and the culture time of the cells is preferably 3 days.

6. A method according to any one of claims 1-5, wherein the medium used in Step 5) is a DMEM/F12 mixture medium supplemented with 5-15% of serum, and contains 5-20 ng/ml of brain-derived neurotrophic factor (BDNF), 10-50 ng/ml of glial cell-derived neurotrophic factor (GDNF) and 20-50 ng/ml of insulinoid growth factor (IGF), and the culture time of the cells is 5-10 days; preferably, the medium is a DMEM/F12 mixture medium supplemented with 10% FBS, and contains 10 ng/ml of BDNF, 20 ng/ml of GDNF and 50 ng/ml of IGF-II, and the culture time of the cells is preferably 7 days.

7. A method according to any one of claims 1-6, the serum is selected from human umbilical cord blood serum and/or fetal bovine serum; the condition of the incubator is 5% carbon dioxide and 95% air.

8. Cells with neuron functions as prepared by the method of any one of claims 1-7, wherein the cells are preferably nerve cells expressing neuron specific enolase (NSE) and neurofilament protein-M (NF-M), and secreting neurotrophic factor (NT) and brain derived neurotrophic factor (BDNF); further preferably the cells are nerve cells expressing neuron specific enolase (NSE), glial fibrillary acidic protein (GFAP), growth related protein factor (GAP-43) and neurofilament protein-M (NF-M), and secreting neurotrophic factor-3 (NT-3), neurotrophic factor-4 (NT-4) and brain derived neurotrophic factor (BDNF).

9. The use of the method in any one of claims 1-7 and the cells with neuron functions in claim 8 in the preparation of medicines for treating injuries with the loss of neuron function;
preferably, the cells with neuron functions are nerve cells expressing neuron specific enolase (NSE), neurofilament protein-M (NF-M), and secreting neurotrophic factor (NT) and brain derived neurotrophic factor(BDNF);
preferably, the injuries with the loss of neuron function are Parkinson's disease and spinal cord injury.

10. The use of the method in any one of claims 1-7 and the cells with neuron functions in claim 8 in the treatment of injuries with the loss of neuron function;
preferably, the injuries are Parkinson's disease and spinal cord injury.

11. A method of treating injuries with the loss of neuron function, wherein the method comprises transplanting the cells with neuron functions produced by the method of claim 8 into the injury sites of patients with neurological diseases, and the neurological diseases include, but not limited to Parkinson's disease and spinal cord injury;
preferably, the cells with neuron functions express neuron specific enolase (NSE) and neurofilament protein-M (NF-M), and secret neurotrophic factor (NT) and brain derived neurotrophic factor(BDNF).
